# EUROPEAN PATENT SPECIFICATION

(11) **EP 1 075 848 B1**
(45) Date of publication and mention of the grant of the patent: **16.11.2005**
(21) Application number: 00116412.8
(22) Date of filing: 28.07.2000
(51) Int. Cl.: A61M 16/06, A61M 16/08, A61M 16/04, A62B 9/06

(54) **Mouthpiece with bendable extra-oral sealing means**
Mundstück mit extraoralen biegsamen Dichtungsmitteln
Embout buccal avec moyens d'étanchéité extra-oraux pliables

(30) Priority: 10.08.1999 AU 4345899; 29.09.1999 NZ 50000099
(43) Date of publication of application: 14.02.2001
(73) Proprietor: Fisher & Paykel Healthcare Limited, East Tamaki, Auckland (NZ)
(72) Inventor: Robertson, Christopher John, Dunedin (NZ); Smith, Nicholas Charles Alan, Auckland (NZ); Gradon, Lewis George, Howick, Auckland (NZ)
(74) Representative: Brown, John D.

(56) References cited:
- EP-A- 0 845 277
- WO-A-90/03199
- WO-A-96/03173
- FR-A- 2 180 022
- US-A- 3 139 088
- US-A- 3 670 726
- US-A- 4 516 573

## Description

This invention relates to a novel mouthpiece for the oral delivery of air in continuous positive airway pressure (CPAP) treatments of sleeping disorders such as sleep apnoea.

### Description of the Prior Art

Sleep apnoea treatments have been significantly advanced with the introduction of continuous positive airway pressure (CPAP) treatments. These treatments, as introduced, involve the supply of gases from a gases supply or blower to a patient through a conduit and nasal mask to provide an elevated internal pressure in the users airways to assist the muscles to keep the airways open. This airstream is provided to the user through a nasal mask applied over the nose and held in place by a harness. This configuration has been almost universally adopted based on the well known observation that humans show a decided preference for nasal breathing during sleep. For this reason, little development has been undertaken into other possible methods of providing the pressurized airstream to a user.

Oral delivery is suggested in EP 818, 213, which shows an apparatus for oral delivery of air in a CPAP treatment. The apparatus includes a mouthpiece adapted to fit inside the mouth between the roof of the mouth, the hard palate, and the tongue, and having a periphery which can be gripped between the teeth. It is thought by the applicants that this is significantly more intrusive than is necessary and is liable to movement and consequent discomfort (although not outright removal) under the relaxation of sleep. It has the additional disadvantage that with the user fully relaxed, such as in the case of sleep, a distension in the user's jaw and subsequent opening of the mouth can reduce the sealing effectiveness of the mouthpiece and reduce the efficacy of the CPAP treatment.

Because the mouthpiece in EP 818,213 is gripped between the user's teeth, a further disadvantage results in that the mouthpiece requires custom orthodontic fitting to ensure that the mouthpiece matches the user's mouth and teeth layout. Custom orthodontic fitting is time consuming and removes the capability of effective mass manufacture. Consequently, the mouthpiece in EP 818,213 is expensive, creating a significant barrier to the patient adoption of the device.

A similar gases delivery mouthpiece, for use with a respirator, is shown in WO 90/03199. WO 90/03199 discloses an orthodontic device which is adapted to be gripped between the jaws of a user and to accommodate the user's teeth within a series of upper and lower cavities. A base member of the mouthpiece is shaped and fits against the hard palate of the user. This mouthpiece again has the disadvantage of requiring custom orthodontic fitting. Furthermore, as a result of the mouthpiece's substantial thickness and size, the mouthpiece is substantially rigid in the vestibule regions of the mouth. The mouthpiece is clamped in place by an outer shield which engages the outside of the user's lips.

A paper by E Veres entitled "Clinical trial of an oral vestibular shield for the control of snoring" (Journal of the Dental Association of South Africa, January 1993) describes the use of a shield intended to be retained in the vestibule of the mouth to seal the mouth and to promote nasal breathing which has been conventionally considered to be more beneficial than oral breathing. Humidified CPAP treatments delivered orally, however, actually derive greater benefit than those delivered nasally because secondary leakage through the nasal passages during oral delivery is significantly less than oral leakage during nasal delivery. The shield depicted in the paper is formed from flexible ethylene vinyl. The shield is custom trimmed and is custom fitted by heating to a malleable temperature and deformed by applied pressure.

Other possible mouthpiece designs are shown for example by use in self contained underwater breathing apparatus systems, for example as depicted in United States Patent No. 4,862,909. This mouthpiece is a mouth guard type and is clamped between the teeth. A flange extends both in front of and behind the teeth.

US3,139,088 discloses an oral inhaler and mouthpiece having a pair of lip sealing plates. The plates engage with a patient's lips when adjusted along the mouthpiece.

US3,882,860 discloses a resuscitation device having two flanges. One of the flanges is laid between the teeth and lips of the patient and the other is slideably arranged to press and fixed on the patient's lips by means of a clamping or arresting device.

Prior art mouthpieces are not well adapted for use in CPAP treatments because they are intended for conscious gripping by the user, and have been found subject to accidental removal with a user in a completely relaxed state such as sleep. The present invention overcomes this problem and present several other advantages which will become apparent upon a reading of the attached specification, in combination with a study of the drawings.

### SUMMARY OF THE INVENTION

It is an object of the present invention to provide a mouthpiece for oral delivery of gases, which goes some way toward overcoming the above disadvantages or which will at least provide the public with a useful choice.

The present invention consists in a mouthpiece as defined in claim 1.

The mouthpiece of the invention comprises:
a vestibular shield having an inner surface and an outer surface, said vestibular shield having a predetermined height which will overlap a user's teeth and gums when positioned in the mouth vestibule of a user;
gases passageway means extending from said outer surface of said vestibular shield to said inner surface of said vestibular shield for allowing the passage of said gases through said mouthpiece; and
extra-oral sealing means associated with said gases passageway or said vestibular shield said extra-oral sealing means capable of being flexed into one of two configurations, a first condition when said mouthpiece is inserted into a user's mouth being substantially unengaged with a user's face, and a second condition when correctly positioned in a user's mouth being substantially engaged with a user's face and under compression thereupon.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting.

The invention consists in the foregoing and also envisages constructions of which the following gives examples.

### BRIEF DESCRIPTION OF THE DRAWINGS

One preferred form of the present invention will now be described with reference to the accompanying drawings in which;
Figure 1 is a side elevational view of a system which may be used by a patient,
Figure 2 is a perspective view from above of a mouthpiece according to the preferred embodiment of the present invention,
Figure 3 is a perspective view from one side and from an inward direction of the mouthpiece of Figure 2,
Figure 4 is a cross-section of the mouthpiece of Figure 2,
Figure 5 is a cross-sectional view of the mouthpiece of Figure 2 and a user with the mouthpiece in place to demonstrate the location and positioning thereof in relation to the main features of the user's anatomy,
Figure 6 is a perspective view of the mouthpiece with the outer flap in place,
Figure 7 is a perspective view of the outer flap bent back,
Figure 8 is a cutaway view of the present invention with the outer flap in use,
Figure 9 is a perspective view of the outer flap including the ventilation apertures and moisture barrier, and
Figure 10 is a block diagram of a respiratory system according to the preferred embodiment of the present invention.

### DETAILED DESCRIPTION OF THE PREFERRED EMBODIMENTS

While the invention may be susceptible to embodiment in different forms, there is shown in the drawings, and herein will be described in detail, specific embodiments with the understanding that the present disclosure is to be considered an exemplification of the principles of the invention, and is not intended to limit the invention to that as illustrated and described herein.

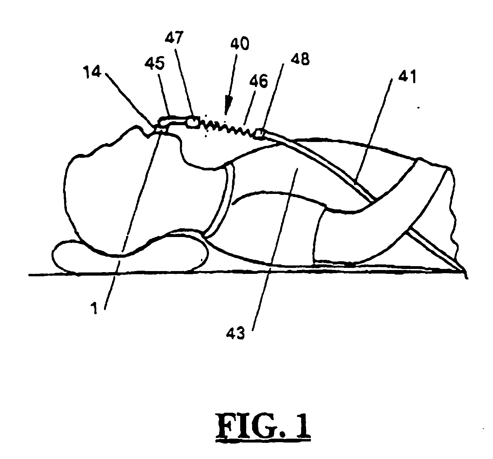

The present disclosure describes a system for oral delivery of gases pressurised above ambient to a user and is especially suited for use in the oral delivery of air in continuous positive airway pressure (CPAP) treatments of sleeping disorders such as sleep apnoea. As shown in Figure 1, the system includes a mouthpiece which is connected by a connection 40 to a breathing circuit 41.

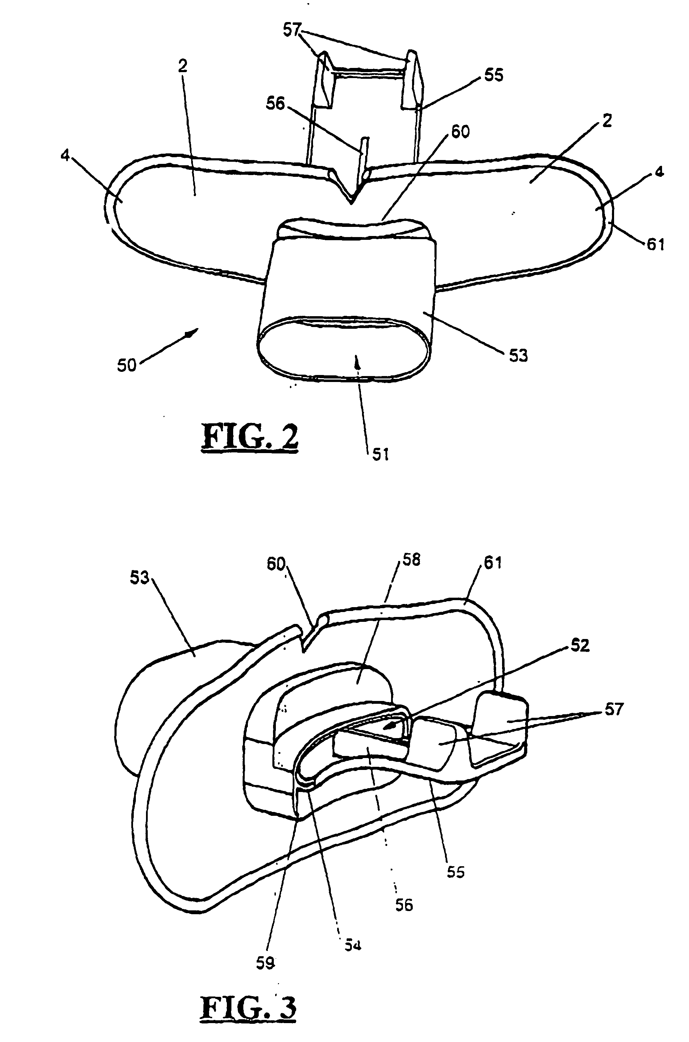

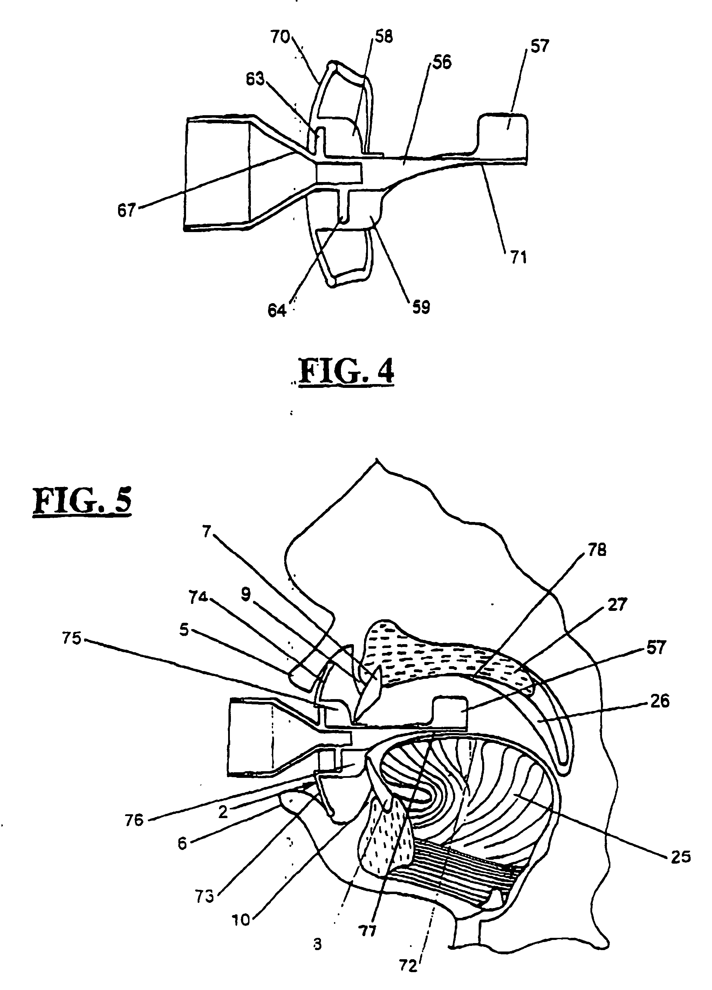

A preferred embodiment of the present invention is illustrated in Figures 2 to 5. In this embodiment, the mouthpiece 50 includes a vestibular shield 2 being a generally flat and generally rectangularly-shaped member in front elevation having a curved profile that reflects the curvature of a user's jaw and in turn the curvature of the labial vestibule region. A gases passageway extends through the vestibular shield from an inlet 51 to an outlet 52 in much the same way as with the earlier embodiments. In the preferred embodiment the inlet 51 is provided by a flattened oval-shaped connector 53. The outlet 52 has an even more laterally extended flattened oval shape 54. The major differences between the mouthpiece 50 and the embodiments described above are provided on the inner face of the vestibular shield. Most prominently, the mouthpiece 50 includes a tongue depressor 55 extending from the inner face of the vestibular shield 2. The operation of the tongue depressor will be described further on with reference to Figure 5. The tongue depressor includes a vertical stiffening flange 56 centrally located on its upper surface and extending from the gases outlet 52. In use gases flow easily around the stiffening flange 56 effectively bifurcating the gases outlet 52. The tongue depressor 55 further includes a pair of vertically extending spacers 57 which in use may abut against the roof of the wearer's mouth and ensure that the tongue cannot completely block the air passageway. In the mouthpiece 50 the sealing effect of the vestibular shield 2 against the lips of the user is enhanced by providing teeth abutments of significantly increased thickness than the raised area 20 of the earlier embodiments. In particular, an upper teeth abutment 58 and a lower teeth abutment 59 are provided, with the lower teeth abutment 59 protruding further from the inner face of the vestibular shield 2 than the upper teeth abutment 58. This difference serves to match the typical over-bite of most users. The abutments 58 and 59 are not required to be wider than the gases outlet 52.

A notch 60 is provided centrally in the upper edge of the vestibular shield 2 to accommodate the upper frenal attachment. A slight bead 61 is provided around the edge of the vestibular shield 2 for user comfort, with the vestibular shield 2 otherwise being very thin for additional suppleness.

Referring particularly to Figure 4, in its preferred form the mouthpiece 50 is preferably formed by over-moulding a soft and supple material part 70 over a stiffer material part 67. These can generally be termed the shield part and the passageway-forming insert. The passageway-forming insert preferably includes a pair of upper and lower vertical flanges 63 and 64 to fully engage within the supple material. The passageway-forming insert 67 includes the vertically extending stiffening flange 56 of the tongue depressor 55, together with a curved planar portion 71 forming the backbone of the tongue depressor 55. The vertically extending spacers 57 are of the soft and supple material and are part of the over-moulding 70, as are the upper and lower teeth abutments 58 and 59.

Referring now to Figure 5, use of the mouthpiece according to Figures 2 to 4 is depicted. With the present mouthpiece 50, the upper and lower lips 5, 6 are further distended by the abutment action of the abutments 75, 76 against the upper and lower teeth 7, 8 respectively, thus forming a seal of greater pressure between the lips 5, 6 and the upper and lower portions respectively of the vestibular shield 2. A lower face 77 of the tongue depressor 55 impinges if necessary on the upper surface 72 of the tongue 25 and retains the tongue in the lower portion of the mouth. This ensures a clear gases outlet 52 from the gases passageway through the vestibular shield. The vertically extending spacers 57, if forced by pressure from the tongue, will engage against the roof of the user's mouth and maintain a clear air passageway. This stops the sleeping patient unconsciously blocking the oral passageway and reverting to nasal breathing.

Attention is now directed to Figure 1. It has been found that an additional factor in the effectiveness of any mouthpiece, including mouthpiece 1, is the manner in which the mouthpiece is connected to the breathing circuit 41. The weight of the breathing circuit 41, and any attempted movement of one other of the breathing circuit 41 and the mouthpiece 1 relative to the other, is one of the largest influences tending to dislodge a mouthpiece 1 from the mouth of a user. It must be noted that the mouthpiece 1 must remain in position and maintain a seal during all sleep, when the user has no muscle tone.

The connection 40 as provided in the present invention between the breathing circuit 41 and the mouthpiece 1 decouples the mouthpiece 1 from the breathing circuit 41. As a result, the connection 40 is effective in reducing the forces placed on the mouthpiece 1 by the breathing circuit 41 when the user moves around during sleep. In the preferred sleeping position, the breathing circuit 41 is laid across the chest 43 of the user, and may be secured to the user's bed clothes or sleeping garments. The breathing circuit 41 is preferably laid on the chest of the user to take the weight of the breathing circuit 41 off of the mouthpiece 1.

To connect between the gases outlet 14 which is vertical when the user is laying on his or her back and the breathing circuit 41 which is generally horizontal, an L-shaped elbow 45 is incorporated in the connection 40. The elbow 45 may be incorporated in the mouthpiece 1, however, it is preferred that the mouthpiece 1 be kept small to provide for easier cleaning. The elbow 45 is formed at a right angle and provides a positive pressure on the mouthpiece 1 to maintain the mouthpiece 1 in the user's mouth. The elbow 45 may include a swivel joint and may be disconnected from gaseous outlet 14. The connection 40 further includes an extremely flexible connecting tube 46 provided between the elbow 45 and the breathing circuit 41. The connecting tube 46 is preferably connected to the breathing circuit 41 by a swivel joint 48 for reasons described herein. The breathing circuit 41, while flexible, will necessarily be stiff enough to maintain its integrity over comparatively long tuns, while the connecting tube 46, being only a short length, for example 10 centimetres, merely has to span between the user's mouth and chest, and can thereby be made in a manner that would not be suitable for long runs. Furthermore, as a result of the short length of the connecting tube 46, the connecting tube 46 does not need to incorporate significant insulation or heating capability. The connecting tube 46 may be formed from a thin plastic membrane supported over a helical or double helical or corrugated supporting ribs. In such a case, the support makes the connection tube 46 laterally flexible and resistant to torsion. The elbow swivel joint 45 allows for movement of the connection tube 46 relative to the mouthpiece 1. The swivel joint 48 allows for movement of the connection tube 46 relative to the breathing circuit 41. It is to be understood that one or both of the swivel joints 45, 48 could be eliminated, but the preferred embodiment includes swivel joint 48.

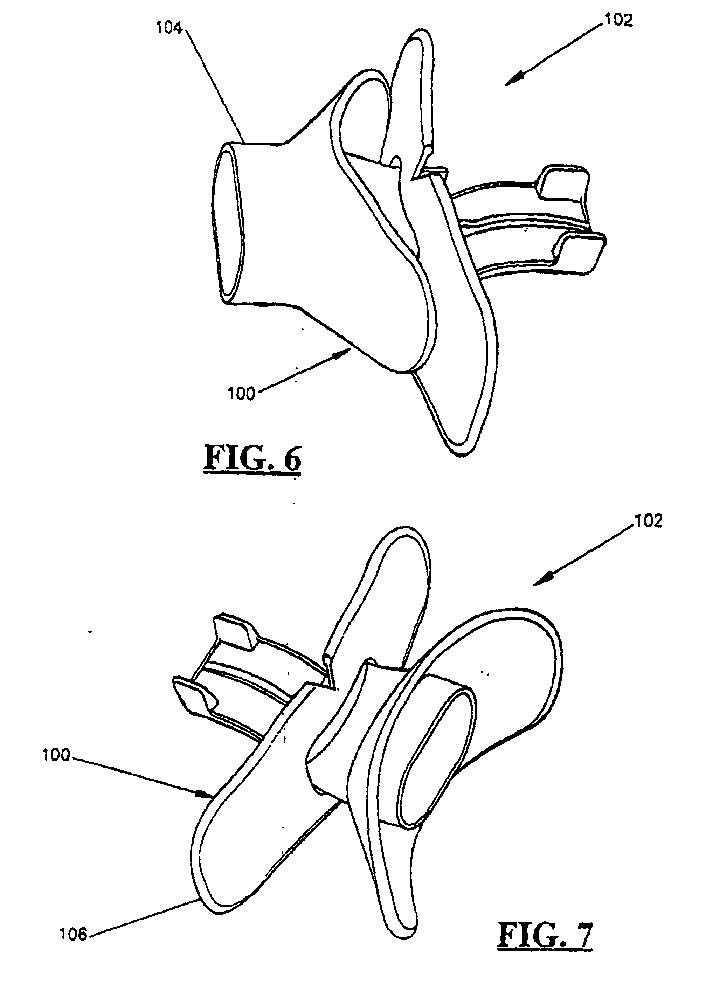

Referring now to Figure 6 of the present invention is illustrated including an extra-oral sealing flap 100. The flap 100 in its natural bias is tapered, the wide open end of which is shaped to conform to the facial contours around the outside of the mouth of a user. The narrow end joins to a cylindrical section, which is designed to slide over the inlet port 104 of the mouthpiece 102. While this is one method of attachment the flap 100 might also be constructed as an integral part of the mouthpiece 102. The flap 100 needs to be constructed of flexible material, therefore materials such as silicone rubber can be employed to fashion the flap.

The outer flap 100 is seen in Figure 7, in a bent back position. It will be appreciated that when the mouthpiece 102 is being inserted into the mouth of a user, the outer flap 100 is intended to be in this bent back position to aid insertion. Prior to insertion, the outer flap is bent back by simply pressing on its outer periphery 106, until it snaps into the bent back position, in which it will stay unaided.

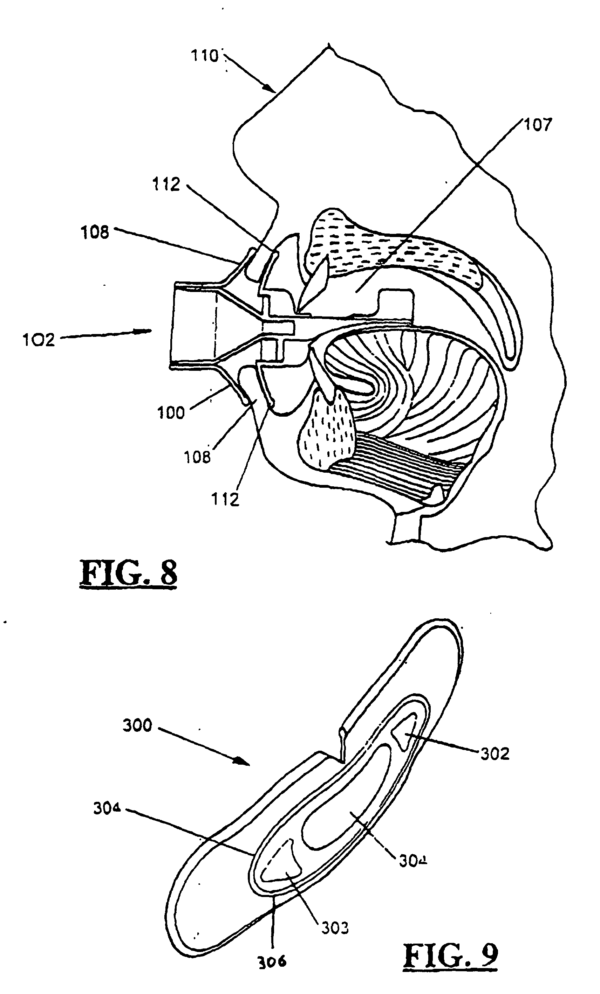

In Figure 8 we see the outer flap 100 in use with the mouthpiece 102 in the mouth 107 of a user 110. Once correctly positioned in the mouth 106, the outer flap 100 may be adjusted into its operational position by pressing on its outer periphery 106 until it snaps back to press against the outside of the mouth 108. Due to the relative position of the vestibular shield 112 and the outer flap 100, the outer flap 100 is unable to fully reach its natural bias and thereby inflicts a compressive force on the outside of the mouth 108.

It will be appreciated that as well as providing a substantially airtight seal the addition of the outer flap provides enough compressive force on the mouth to keep the mouthpiece and conduit in place without the need for straps. This allows the administering of CPAP therapy to be considerably less obtrusive than traditional methods.

In a further additional improvement shown in Figure 9, the outer flap 300 is shown in perspective. Included are ventilation apertures 302, 303 either side of the gases port 304, which are surrounded by a ridge 306 acting as a moisture barrier. The apertures 302,303 are provided such that any excess moisture leaking from the mouth will migrate to the apertures where they may evaporate. Small vents in the conduit may be used to direct small amounts of pressurised gas at the apertures to aid evaporation. The ridge 306 is included to ensure that no moisture migrates further into the sealing region 308, as this would be detrimental to the sealing properties of the flap.

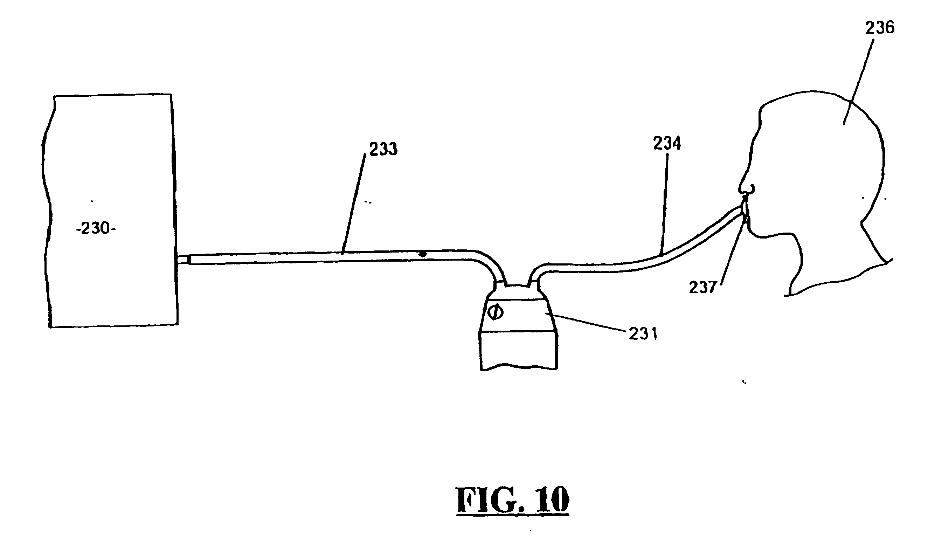

A typical respiratory humidification circuit such as might employ the present invention is shown diagrammatically in Figure 10, and includes the respirator 230, humidifier 231, and the associated respiratory breathing tubes 233 and 234. A patient 236 under treatment is shown, with the present invention 237, located in the mouth of the patient 236.

From the above it can be seen that the present invention provides a mouthpiece 1 for oral delivery of CPAP treatment which at once is low cost and effective. Unlike other appliances the mouthpiece 1 used in the present invention does not require custom orthodontic fitting as the mouthpiece 1 does not rely on accurate alignment with the user's teeth or the user's palate to provide location and retention within the user's mouth, but instead resides in the vestibule between the teeth and lips and the teeth and cheeks, and the lateral and vertical extension of the vestibular shield 2 requires that the user's lips be actively manipulated for the vestibular shield 2 to be removed. Furthermore the improved connection 40 to the breathing circuit 41 reduces the forces which tend to pull at the mouthpiece 1. With the addition of the extra-oral flap 100, the mouthpiece and associated tubing is held securely in place without the need for external strapping, and an effective seal is created around the users mouth.

## Claims

1. A mouthpiece (102) for delivering gases to the oral cavity of a user (110) comprising:
a vestibular shield (112) having an inner surface and an outer surface, said vestibular shield (112) having a predetermined height which will overlap a user's teeth and gums when positioned in the mouth vestibule of a user;
gases passageway means (53) extending from said outer surface of said vestibular shield (112) to said inner surface of said vestibular shield (112) for allowing the passage of said gases through said mouthpiece (102); and
extra-oral sealing means (100) associated with one of said gases passageway (53) and said vestibular shield (112) **characterised in that** said extra-oral sealing means (100) is capable of being flexed into one of two configurations, a first condition when said mouthpiece (102) is inserted into a user's mouth with said extra-oral sealing means substantially unengaged with a user's face, and a second condition when correctly positioned in a user's mouth with said extra-oral sealing means (112) substantially engaged with a user's face and under compression thereupon.

2. A mouthpiece as claimed in claim 1 wherein said mouthpiece (102) further includes a central portion which will extend over a user's front teeth and gums when said central portion is positioned between the lips and teeth of said user, and outer portions (4) extending from said central portion which extend along and overlap at least a portion of the user's back teeth and gums when said outer portions are postioned between the cheeks and teeth of the user and a raised portion (58) provided on said inner surface of said central portion of said vestibular shield, said raised portion solely contacting the teeth of a user when said vestibular shield (112) is placed in the mouth of a user.

3. A mouthpiece as claimed in claim 2 wherein said raised portion (58) is of a generally flattened oval shape.

4. A mouthpiece as claimed in claim 2 wherein said raised portion (58) extends along at least a portion of said outer portions (4).

5. A mouthpiece as claimed in claim 1 wherein said central portion includes a centrally located notch (60) in a top edge thereof.

6. A mouthpiece as claimed in claim 1 wherein said vestibular shield (112) has a generally curved profile.

7. A mouthpiece as claimed in claim 1 wherein said gases passageway means (53) includes gases inlet means (51) for allowing connecting of said mouthpiece (102) to a gases supply (230).

8. A mouthpiece as claimed in claim 1 wherein said gases inlet means (51) is connected to said central portion of said vestibular shield (112).

9. A mouthpiece as claimed in claim 1 wherein said vestibular shield (112) is formed of a supple material and said gases inlet means (51) comprises a tube formed of a stiffer material than said supple material that is used to form said vestibular shield, said tube being attached to said vestibular shield.

10. A mouthpiece as claimed in claim 1 wherein said gases passageway means (53) includes gases outlet means (52) for allowing gases to be expelled into the mouth of a user.

11. A mouthpiece as claimed in claim 10 wherein said gases outlet means (52) is provided along said central portion of said vestibular shield (112).

12. A mouthpiece as claimed in claim 11 wherein said gases passageway means (53) includes gases inlet means (51) for allowing connection of said mouthpiece to a gases supply.

13. A mouthpiece as claimed in claim 12 wherein said gases outlet means (52) is aligned with said gases inlet means (51).

14. A mouthpiece as claimed in claim 13 wherein said gases inlet means (51) and said gases outlet means (52) are connected to said central portion of said vestibular shield (112).

15. A mouthpiece as claimed in claim 14 wherein said gases outlet means (52) is a generally flattened oval-shaped tube which extends from said inner surface of said vestibular shield (112).

16. A mouthpiece as claimed in claim 15 wherein said outer portions (4) of said vestibular shield (112) include a plurality of ribs thereon.

17. A mouthpiece as claimed in claim 1 wherein said ribs are provided on said outer surface of said vestibular shield (112).

18. A mouthpiece as claimed in claim 1 wherein said vestibular shield (112) is formed of a supple material.

19. A mouthpiece as claimed in claim 1 wherein said vestibular shield (112) is generally flat.

20. A mouthpiece as claimed in claim 1 wherein each said outer portion (4) has rounded upper and lower edges.

21. A mouthpiece as claimed in claim 2 wherein said raised portion (58) includes an upper abutment portion (58) for abutting against the upper teeth and a lower abutment portion (59) for abutting against the lower teeth and the lower abutment portion (59) is raised further from the inner surface of the vestibular shield (112) than said upper abutment portion (58).

22. A mouthpiece as claimed in claim 1 including a tongue depressing guard (55) extending from the inner side of said vestibular shield (112).

23. A mouthpiece as claimed in claim 22 wherein said tongue depressor (55) has a concave curve in its lower surface and is stiffer than the outer portions (4) of said vestibular shield (112).

24. A mouthpiece as claimed in claim 23 wherein said mouthpiece (102) including one or more vertically extending spacers (57) projecting from the upper surface of said tongue depressing guard (55).

25. A mouthpiece as claimed in claim 24 wherein said gases passageway (53) is provided by an insert of substantially stiffer material than the material of said vestibular shield (112), said insert extending through said vestibular shield, and said tongue depressing guard (55) includes therein an extension of said insert forming a stiff backbone (71) therefor.

26. A mouthpiece as claimed in claim 1 wherein said extra-oral sealing means (100) are detachable from said gases passageway (53).

27. A mouthpiece as claimed in claim 1 wherein said extra-oral sealing means (100) are constructed of silicon rubber.

28. A mouthpiece as claimed in claim 1 wherein said extra-oral sealing means (100) comprise at least one tapered flap (100).

29. A mouthpiece as claimed in claim 28 wherein said flap (100) has a wide end and a narrow end, said narrow end being attached to said gases passageway (53).

30. A mouthpiece as claimed in claim 29 wherein said first condition comprises said wide end being distal to a user relative to said narrow end being proximal to a user.

31. A mouthpiece as claimed in claim 30 wherein said second condition comprises said wide end being proximal to a user relative to said narrow end being distal to a user.

32. A mouthpiece as claimed in claim 1 wherein the compressive force between said vestibular shield (112) and said extra-oral sealing means (100) on the area surrounding a user's lips is sufficient to secure said mouthpiece (102) in place on a user and to provide a substantial seal thereto.

33. A mouthpiece as claimed in claim 29 wherein said wide end is adapted to conform to the facial contours of a user.

34. A mouthpiece as claimed in claim 28 wherein said flap (100) includes at least one ventilation means (303), proximal to said narrow end.

35. A mouthpiece as claimed in claim 34 wherein said narrow end and said at least one ventilator means (303) are surrounded by a ridge (306) on the side of said flap (100) which in use faces a user.

36. A mouthpiece as claimed in claim 34 wherein said at least one ventilator means (303) comprises two apertures either side of said narrow end.

37. A mouthpiece as claimed in claim 36 wherein said mouthpiece includes means which are adapted to direct a small amount of pressurised gases in the vicinity of said apertures.

## Patentansprüche

1. Mundstück (102) für die Zufuhr von Gasen zur Mundhöhle eines Benutzers (110), umfassend:
eine Mundvorhof-Schutzabdeckung (112) mit einer Innenfläche und einer Außenfläche, wobei die Mundvorhof-Schutzabdeckung (112) eine vorgegebene Höhe hat, die bei Positionierung der Schutzabdeckung in dem Mundvorhof eines Benutzers Zähne und Zahnfleisch des Benutzers überlappt;
Gasdurchlassmittel (53), die sich von der Außenfläche der Mundvorhof-Schutzabdeckung (112) zur Innenfläche der Mundvorhof-Schutzabdeckung (112) erstrecken, um die Gase durch das Mundstück hindurch (102) passieren zu lassen; und
extraorale Abdichtungsmittel (100), die von dem Gasdurchlass (53) und der Mundvorhof-Schutzabdeckung (112) einem/einer zugeordnet sind,
**dadurch gekennzeichnet,**
**dass** das extraorale Abdichtungsmittel in eine von zwei Konfiguration gebogen werden kann, wobei ein erster Zustand ist, dass das Mundstück (102) in den Mund eines Benutzers eingesetzt ist und dabei das extraorale Abdichtungsmittel im wesentlichen nicht an dem Gesicht eines Benutzers anliegt, und wobei ein zweiter Zustand ist, dass bei korrekter Positionierung des Mundstücks in dem Mund eines Benutzers das extraorale Abdichtungsmittel (100) im wesentlichen an dem Gesicht eines Benutzers anliegt und an das Gesicht gedrückt wird.

2. Mundstück nach Anspruch 1, wobei das Mundstück (102) femer einen zentralen Bereich aufweist, der sich bei seiner Positionierung zwischen den Lippen und den Zähnen des Benutzers über die Vorderzähne und das Zahnfleisch des Benutzers erstreckt, und sich von dem zentralen Bereich erstreckende äußere Bereiche (4), die sich über zumindest einen Teil der hinteren Zähne und des hinteren Zahnfleisches erstrecken und diesen zumindest einen Teil der hinteren Zähne und des hinteren Zahnfleisches überlappen, wenn die äußeren Bereiche zwischen den Backen und den Zähnen des Benutzers positioniert sind, und einen an der Innenfläche des zentralen Bereichs der Mundvorhof-Schutzabdeckung vorgesehenen erhabenen Bereich (68), der allein mit den Zähnen des Benutzers in Kontakt ist, wenn die Mundvorhof-Schutzabdeckung (112) in dem Mund des Benutzers platziert ist.

3. Mundstück nach Anspruch 2, wobei der erhabene Bereich (58) eine allgemein abgeflachte ovale Form hat.

4. Mundstück nach Anspruch 2, wobei sich der erhabene Bereich (58) entlang wenigstens eines Teils der äußeren Bereiche (4) erstreckt.

5. Mundstück nach Anspruch 1, wobei der zentrale Bereich in seiner Oberkante eine zentral angeordnete Kerbe (60) aufweist.

6. Mundstück nach Anspruch 1, wobei die Mundvorhof-Schutzabdeckung (112) ein allgemein gebogenes Profil hat.

7. Mundstück nach Anspruch 1, wobei das Gasdurchlassmittel (53) ein Gaseinlassmittel (51) aufweist, das die Verbindung des Mundstücks (102) mit einer Gasversorgung (230) erlaubt.

8. Mundstück nach Anspruch 1, wobei das Gaseinlassmittel (51) mit dem zentralen Bereich der Mundvorhof-Schutzabdeckung (112) verbunden ist.

9. Mundstück nach Anspruch 1, wobei die Mundvorhof-Schutzabdeckung (112) aus einem nachgiebigen Material gebildet ist und das Gaseinlassmittel (51) ein Rohr umfasst, das aus einem Material gebildet ist, das steifer ist als das für die Bildung der Mundvorhof-Schutzabdeckung verwendete nachgiebige Material, wobei das Rohr an der Mundvorhof-Schutzabdeckung angebracht ist.

10. Mundstück nach Anspruch 1, wobei das Gasdurchlassmittel (53) ein Gasauslassmittel (52) aufweist, um einen Ausstoß von Gasen in den Mund eines Benutzers zu ermöglichen.

11. Mundstück nach Anspruch 10, wobei das Gasauslassmittel (52) entlang des zentralen Bereichs der Mundvorhof-Schutzabdeckung (112) vorgesehen ist.

12. Mundstück nach Anspruch 11, wobei das Gasdurchlassmittel (53) ein Gaseinlassmittel (51) aufweist, um eine Verbindung des Mundstücks mit einer Gasversorgung zu ermöglichen.

13. Mundstück nach Anspruch 12, wobei das Gasauslassmittel (52) auf das Gaseinlassmittel (51) ausgerichtet ist.

14. Mundstück nach Anspruch 13, wobei das Gaseinlassmittel (51) und das Gesauslassmittel (52) mit dem zentralen Bereich der Mundvorhof-Schutzabdeckung (112) verbunden sind.

15. Mundstück nach Anspruch 14, wobei das Gasauslassmittel (52) ein allgemein abgeflachtes oval geformtes Rohr ist, das sich von der innenfläche der Mundvorhof-Schutzabdeckung (112) erstreckt.

16. Mundstück nach Anspruch 15, wobei die äußeren Bereiche (4) der Mundvorhof-Schutzabdeckung (112) eine Mehrzahl von an ihnen gebildeten Rippen aufweisen.

17. Mundstück nach Anspruch 1, wobei die Rippen an der Außenfläche der Mundvorhof-Schutzabdeckung (112) vorgesehen sind.

18. Mundstück nach Anspruch 1, wobei die Mundvorhof-Schutzabdeckung (112) aus einem nachgiebigen Material gebildet ist.

19. Mundstück nach Anspruch 1, wobei die Mundvorhof-Schutzabdeckung (112) allgemein flach ist.

20. Mundstück nach Anspruch 1, wobei jeder der äußeren Bereiche (4) gerundete Ober- und Unterkanten hat.

21. Mundstück nach Anspruch 2, wobei der erhabene Bereich (58) einen oberen Anlagebereich (58) für die Anlage an den oberen Zähnen und einen unteren Anlagebereich (59) für die Anlage an den unteren Zähnen umfasst und wobei der untere Anlagebereich (59) von der Innenfläche der Mundvorhof-Schutzabdeckung (112) weiter erhaben ist als der obere Anlagebereich (58).

22. Mundstück nach Anspruch 1, umfassend einen Zungen-Niederhaltewächter (55), der sich von der Innenselte der Mundvorhof-Schutzabdeckung (112) erstreckt.

23. Mundstück nach Anspruch 22, wobei der Zungen-Niederhalter (55) in seiner Unterfläche eine konkave Krümmung aufweist und steifer ist als die äußeren Bereiche (4) der Mundvorhof-Schutzabdeckung (112).

24. Mundstück nach Anspruch 23, wobei das Mundstück (102) einen oder mehrere sich vertikal erstreckende Abstandshalter (57) aufweist, die von der Oberfläche des Zungen-Niederhaltewächters (55) abragen.

25. Mundstück nach Anspruch 24, wobei der Gasdurchlass (53) bereitgestellt ist durch einen Einsatz aus einem Material, das wesentlich steifer ist als das Material der Mundvorhof-Schutzabdeckung (112), wobei sich der Einsatz durch die Mundvorhof-Schutzabdeckung hindurch erstreckt und der Zungen-Niederhaltewächter (55) einen Fortsatz des Einsatzes umfasst, der ein steifes Rückgrat (71) dafür bildet.

26. Mundstück nach Anspruch 1, wobei die extraoralen Abdichtungsmittel (100) von dem Gasdurchlass (53) abnehmbar sind.

27. Mundstück nach Anspruch 1, wobei die extraoralen Abdichtungsmittel (100) aus Silikongummi hergestellt sind.

28. Mundstück nach Anspruch 1, wobei die extraoralen Abdichtungsmittel (100) zumindest einen konisch zulaufenden Flügel (100) umfassen.

29. Mundstück nach Anspruch 28, wobei der Flügel (100) ein breites Ende und ein schmales Ende besitzt und das schmale Ende an dem Gasdurchlass (53) angebracht ist.

30. Mundstück nach Anspruch 29, wobei der erste Zustand umfasst, dass das breite Ende verglichen mit dem dem Benutzer nahen schmalen Ende das von dem Benutzer feme Ende ist.

31. Mundstück nach Anspruch 30, wobei der zweite Zustand umfasst, dass das breite Ende verglichen mit dem von dem Benutzer femen schmalen Ende das dem Benutzer nahe Ende ist.

32. Mundstück nach Anspruch 1, wobei die Druckkraft zwischen der Mundvorhof-Schutzabdeckung (112) und dem extraoralen Abdichtungsmittel (100) an dem die Lippen eines Benutzers umgebenden Bereich ausreicht, um die Lage des Mundstücks (102) an einem Benutzer zu sichem und für seine wesentliche Abdichtung zu sorgen.

33. Mundstück nach Anspruch 29, wobei das breite Ende derart ausgebildet ist, dass es sich an die Gesichtskonturen eines Benutzers anpasst.

34. Mundstück nach Anspruch 28, wobei der Flügel (100) zumindest ein Ventilationsmittel (303) in der Nähe des schmalen Endes aufweist.

35. Mundstück nach Anspruch 34, wobei das schmale Ende und das zumindest eine Ventilatormittel (303) auf der Seite des Flügels (100), die bei Benutzung dem Benutzer zugekehrt ist, von einem Steg (306) umgeben sind.

36. Mundstück nach Anspruch 34, wobei das zumindest eine Ventilatormittel (303) auf jeder Seite des schmalen Endes zwei Öffnungen aufweist.

37. Mundstück nach Anspruch 36, wobei das Mundstück Mittel aufweist, die derart ausgelegt sind, dass sie eine geringe Menge an Druckgas In die Nähe der Öffnungen leiten.

## Revendications

1. Embout buccal (102) pour délivrer des gaz à la cavité orale d'un utilisateur (110), comprenant :
un écran vestibulaire (112) ayant une surface interne et une surface externe, ledit écran vestibulaire (112) ayant une hauteur prédéterminée qui chevauchera les dents et les gencives d'un utilisateur lorsqu'il est positionné dans le vestibule de la bouche d'un utilisateur;
des moyens de passage de gaz (53) s'étendant de ladite surface externe dudit écran vestibulaire (112) à ladite surface interne dudit écran vestibulaire (112) pour permettre le passage desdits gaz à travers ledit embout buccal (102); et
des moyens d'étanchéité extra-oraux (100) associés à un desdits passages de gaz (53) et audit écran vestibulaire (112), **caractérisé en ce que** lesdits moyens d'étanchéité extra-oraux (100) sont capables de fléchir dans une de deux configurations, une première condition lorsque ledit embout buccal (102) est inséré dans la bouche d'un utilisateur et où lesdits moyens d'étanchéité extra-oraux ne sont sensiblement pas engagés sur le visage d'un utilisateur, et une seconde condition lorsqu'il est positionné correctement dans la bouche d'un utilisateur et où lesdits moyens d'étanchéité extra-oraux (112) sont sensiblement engagés sur le visage d'un utilisateur et sous compression sur celui-ci.

2. Embout buccal selon la revendication 1, dans lequel ledit embout buccal (102) comprend en outre une partie centrale qui s'étendra sur les dents et les gencives de devant d'un utilisateur lorsque ladite partie centrale est positionnée entre les lèvres et les dents dudit utilisateur, et des parties externes (4) s'étendant depuis ladite partie centrale, qui s'étendent le long et recouvrent au moins une partie des dents et des gencives du fond de l'utilisateur lorsque lesdites parties externes sont positionnées entre les joues et les dents de l'utilisateur et une partie surélevée (58) aménagée sur ladite surface interne de ladite partie centrale dudit écran vestibulaire, ladite partie surélevée n'étant en contact avec les dents d'un utilisateur que lorsque ledit écran vestibulaire (112) est placé dans la bouche d'un utilisateur.

3. Embout buccal selon la revendication 2, dans lequel ladite partie surélevée (58) a une forme ovale généralement aplatie.

4. Embout buccal selon la revendication 2, dans lequel ladite partie surélevée (58) s'étend le long d'au moins une partie desdites parties externes (4).

5. Embout buccal selon la revendication 1, dans lequel ladite partie centrale comprend une encoche (60) placée au centre dans un bord supérieur de celle-ci.

6. Embout buccal selon la revendication 1, dans lequel ledit écran vestibulaire (112) a un profil généralement incurvé.

7. Embout buccal selon la revendication 1, dans lequel lesdits moyens de passage de gaz (53) comprennent un moyen d'entrée de gaz (51) pour permettre le raccordement dudit embout buccal (102) à une alimentation en gaz (230).

8. Embout buccal selon la revendication 1, dans lequel ledit moyen d'entrée de gaz (51) est raccordé à ladite partie centrale dudit écran vestibulaire (112).

9. Embout buccal selon la revendication 1, dans lequel ledit écran vestibulaire (112) est formé d'un matériau souple et ledit moyen d'entrée de gaz (51) comprend un tube formé d'un matériau plus rigide que ledit matériau souple qui est utilisé pour former ledit écran vestibulaire, ledit tube étant fixé audit écran vestibulaire.

10. Embout buccal selon la revendication 1, dans lequel lesdits moyens de passage de gaz (53) comprennent un moyen de sortie de gaz (52) pour pouvoir expulser les gaz dans la bouche d'un utilisateur.

11. Embout buccal selon la revendication 10, dans lequel ledit moyen de sortie de gaz (52) est aménagé le long de ladite partie centrale dudit écran vestibulaire (112).

12. Embout buccal selon la revendication 11, dans lequel lesdits moyens de passage de gaz (53) comprennent un moyen d'entrée de gaz (51) pour permettre le raccordement dudit embout buccal à une alimentation en gaz.

13. Embout buccal selon la revendication 12, dans lequel ledit moyen de sortie de gaz (52) est aligné avec ledit moyen d'entrée de gaz (51).

14. Embout buccal selon la revendication 13, dans lequel ledit moyen d'entrée de gaz (51) et ledit moyen de sortie de gaz (52) sont raccordés à ladite partie centrale dudit écran vestibulaire (112).

15. Embout buccal selon la revendication 14, dans lequel ledit moyen de sortie de gaz (52) est un tube de forme ovale généralement aplati qui s'étend depuis ladite surface interne dudit écran vestibulaire (112).

16. Embout buccal selon la revendication 15, dans lequel lesdites parties externes (4) dudit écran vestibulaire (112) portent une pluralité de nervures.

17. Embout buccal selon la revendication 1, dans lequel lesdites nervures sont aménagées sur ladite surface externe dudit écran vestibulaire (112).

18. Embout buccal selon la revendication 1, dans lequel ledit écran vestibulaire (112) est formé d'un matériau souple.

19. Embout buccal selon la revendication 1 dans lequel ledit écran vestibulaire (112) est généralement aplati.

20. Embout buccal selon la revendication 1, dans lequel chaque dite partie externe (4) a des bords supérieur et inférieur arrondis.

21. Embout buccal selon la revendication 2, dans lequel ladite partie surélevée (58) comprend une partie de butée supérieure (58) pour s'abouter contre les dents supérieures et une partie de butée inférieure (59) pour s'abouter contre les dents inférieures et la partie de butée inférieure (59) est plus surélevée par rapport à la surface interne dudit écran vestibulaire (112) que ladite partie de butée supérieure (58).

22. Embout buccal selon la revendication 1, comprenant un abaisse-langue (55) s'étendant depuis le côté interne dudit écran vestibulaire (112).

23. Embout buccal selon la revendication 22, dans lequel ledit abaisse-langue (55) a une courbure concave dans sa surface inférieure et est plus rigide que les parties externes (4) dudit écran vestibulaire (112).

24. Embout buccal selon la revendication 23, dans lequel ledit embout buccal (102) comprenant un ou plusieurs éléments d'espacement (57) s'étendant verticalement et faisant saillie de la surface supérieure dudit abaisse-langue (55).

25. Embout buccal selon la revendication 24, dans lequel ledit passage de gaz (53) est formé par un élément rapporté d'un matériau sensiblement plus rigide que le matériau dudit écran vestibulaire (112), ledit élément rapporté s'étendant à travers ledit écran vestibulaire, et ledit abaisse-langue (55) comprend une extension dudit élément rapporté formant une ossature rigide (71) prévue à cet effet.

26. Embout buccal selon la revendication 1, dans lequel lesdits moyens d'étanchéité extra-oraux (100) sont détachables desdits passages de gaz (53).

27. Embout buccal selon la revendication 1 dans lequel lesdits moyens d'étanchéité extra-oraux (100) sont construits en caoutchouc siliconé.

28. Embout buccal selon la revendication 1, dans lequel lesdits moyens d'étanchéité extra-oraux (100) comprennent au moins un rabat effilé (100).

29. Embout buccal selon la revendication 28, dans lequel ledit rabat (100) a une extrémité large et une extrémité étroite, ladite extrémité étroite étant fixée audit passage de gaz (53).

30. Embout buccal selon la revendication 29, dans lequel ladite première condition comprend ladite extrémité large qui est distale à un utilisateur par rapport à ladite extrémité étroite qui est proximale à un utilisateur.

31. Embout buccal selon la revendication 30, dans lequel ladite seconde condition comprend ladite extrémité large qui est proximale à un utilisateur par rapport à ladite extrémité étroite qui est distale à un utilisateur.

32. Embout buccal selon la revendication 1, dans lequel ladite force compressive entre ledit écran vestibulaire (112) et lesdits moyens d'étanchéité extra-oraux (100) sur la zone entourant les lèvres d'un utilisateur est suffisante pour fixer ledit embout buccal (102) en place sur un utilisateur et lui offrir une étanchéité notable.

33. Embout buccal selon la revendication 29, dans lequel ladite extrémité large est adaptée pour se conformer aux contours faciaux d'un utilisateur.

34. Embout buccal selon la revendication 28, dans lequel ledit rabat (100) comprend au moins un moyen de ventilation (303), proximal à ladite extrémité étroite.

35. Embout buccal selon la revendication 34, dans lequel ladite extrémité étroite et ledit au moins un moyen de ventilation (303) sont entourés d'une nervure (306) sur le côté dudit rabat (100) qui, lors de l'utilisation, est tourné vers un utilisateur.

36. Embout buccal selon la revendication 34, dans lequel ledit au moins un moyen de ventilation (303) comprend deux ouvertures sur l'un ep l'autre côté de ladite extrémité étroite.

37. Embout buccal selon la revendication 36, dans lequel ledit embout buccal comprend des moyens qui sont à même de diriger une petite quantité de gaz sous pression à proximité desdites ouvertures.
